# EUROPEAN PATENT SPECIFICATION

(11) **EP 2 545 954 B1**
(45) Date of publication and mention of the grant of the patent: **11.05.2016**
(21) Application number: 12176323.9
(22) Date of filing: 13.07.2012
(51) Int. Cl.: A61M 25/02

(54) **Catheter fixation device**
Katheterfixierungsvorrichtung
Dispositif de fixation de cathéter

(30) Priority: 13.07.2011 JP 2011155276
(43) Date of publication of application: 16.01.2013
(73) Proprietor: Covidien LP, Mansfield, MA 02048 (US)
(72) Inventor: KAZUHIRO Abe, Shizuoka 437-0004 (JP); KAZUHIRO Koike, Shizuoka 437-0004 (JP); MASANORI Makino, Shizuoka 437-0004 (JP); AKAIKE Yoshimi, Shizuoka 437-0004 (JP); KANIE Nobuatsu, Shizuoka 437-0004 (JP); MIZOGUCHI Masato, Shizuoka 437-0004 (JP)
(74) Representative: Gray, James

(56) References cited:
- EP-A2- 0 327 299
- WO-A1-2010/033858
- JP-A- 8 024 344
- US-A1- 2006 058 738
- US-A1- 2009 093 766

## Description

### BACKGROUND

### 1. Technical Field

The present invention relates to a catheter fixing device that is used when fixing a catheter to the skin of a patient.

### 2. Background of Related Art

Recently, treatments and diagnoses using catheters have been widely implemented in the medical field. Such catheters, when used, require that a catheter tip be reliably indwelled at a site suitable for the treatment or diagnosis, and that a part outside of the body coming out of the skin is generally affixed to the patient.

A catheter fixing device has been conventionally proposed as a means for fixation to the patient, to be used when fixing the catheter to the surface of the body of the patient. This kind of catheter fixing device is provided with, for example, a fixing device body made from a soft resin, and a fixed member made from a metal or resin of a higher rigidity than the fixing device body.

The fixing device body is provided with retaining parts that can be deployed by inserting the catheter. In the state wherein the catheter has been inserted and deployed, nearly the entire circumference of the catheter is covered by the retaining parts. In order to impart an adequate reinforcing effect to the fixing device body, the fixed member is covered from above the fixing device body. As a result, the catheter is made to be reliably fixed by clamping the retaining parts.

However, several other types of catheter fixing devices are known in the prior art. For example, in publication JP-A 2003-154012 and publication JP-A 2008-212434, the fixing device body and the fixed member are each configured separately, but are linked together so as to form an integrated body.

However, conventional catheter fixing devices end up being a burden on the patient, because there tends to be a compressive force applied to the skin when the fixed member is fitted by being covered from the vertical direction of the fixing device body.

Moreover, in the case of this type of catheter fixing device, a clamping part must necessarily be provided in the height direction at a predetermined point in the fixing device body in order to prevent falling out by endowing the fixed member with a certain degree of interlocking force. Therefore, the thickness of the fixing device overall is increased and ends up becoming unwieldy. As a result, a sense of discomfort is experienced at the point of fixation with the catheter fixing device. Also, when the catheter fixing device and the puncture part are plastered with a dressing (medical adhesive tape), air bubbles are prone to form around the unwieldy catheter fixing device, which may become a hotbed for infection.

Moreover, when the dressing is peeled off in the conventional catheter fixing devices described above, the fixed member may be removed from the patient with the dressing.

Also, the conventional catheter fixing devices are, in terms of the structure thereof, without any margin of allowance, such that fixation is often poor and often leads to the catheter falling out.

WO2010/033858 discloses a securement system including a retainer and a clamp which include interengaging structure.

JP8024344 discloses a catheter fixer including a holder with a hole for inserting a catheter and a clip to fix the catheter.

EP0327299 discloses a catheter securing button.

US2006/058738 discloses a catheter clamp.

### SUMMARY

The present invention was made in view of the problems described above, and the objective thereof is to provide a catheter fixing device wherein the total thickness can be held in check while unintentional disconnection can still be prevented, without posing much burden for the patient and despite the ability to be stably fixed.

The following is a list of five (5) catheter fixing device embodiments for solving the above problems. In a first embodiment, a catheter fixing device for fixing a tubular catheter is provided with a plate-shaped fixing device body having retaining parts and a pair of wing pieces that form a catheter retention slot. The catheter fixing device is also provided with a fixed member that can be fitted onto the fixing device body by being slid along the planar direction of the fixing device body. The retaining parts are compressed by the fixed member when the fixed member is fitted so as to narrow the catheter retention slot.

According to the first embodiment of the invention, sliding the fixed member along the planar direction of the fixing device body causes the fixed member to fit onto the fixing device body. The retaining parts of the fixing device body are thereupon compressed by the fixed member, thus narrowing the catheter retaining slot, as a result of which the catheter is clamped and fixed by the retaining parts. According to such a configuration, when the fixed member is slid in so as to be fitted, compressive force is less likely to be applied to the skin in the vertical direction. Accordingly, the catheter can be stably fixed without posing a great burden to the patient. Moreover, when the dressing intended to protect the catheter fixing device is peeled off, accidental events such as the fixed member coming off therewith are less likely to occur, because it is difficult to apply force in the direction that would separate the fixing member. Furthermore, the total thickness of the catheter fixing device can be held in check even when the fixed member is provided with structures such as clamping parts.

In the second embodiment, the surface side of the fixing device body is provided with guiding parts that extend along the planar direction of the fixing device body of the first embodiment, and the fixed member is provided with guided parts that can be locked into the guiding parts.

According to the second embodiment of the invention, because the guided parts can be locked into the guiding parts, the fixed member can be slid smoothly while being guided along the planar direction of the fixing device body. Also, the fixed member is less likely to come off of the fixing device body in the state wherein the fixed member has been fitted into the fixing device body.

In the third embodiment, the catheter fixing device of the second embodiment is provided within catheter retention slot which opens out on the surface side of the fixing device body, and the guiding parts are provided as a left-right pair on the retaining parts, such that the retaining parts are compressed from both the left and the right by the guided parts when the fixed member is fitted so as to narrow the catheter retention slot.

Therefore, according to the third embodiment, the fixed member can be more stably and smoothly slid in since the guiding parts are provided as a left-right pair on the retaining parts.

Additionally, as a result of the fact that the retaining parts are evenly compressed from both the left and right sides by the guided parts of the fixed member when the fixed member is fitted, the entire catheter retaining slot can be reliably narrowed, and the catheter can thus be more stably fixed.

In the fourth embodiment, the catheter fixing device of the third embodiment is provided with a fixed member which is a covering that entirely covers the guiding parts in the state wherein the reverse side thereof is made to contact the surface of the guiding parts.

According to the fourth embodiment, the total thickness of the catheter fixing device can be reliably held in check because the reverse surface of the covering, which is the fixed member, and the upper surface of the guiding parts are in a state of contact. Furthermore, by entirely covering the guiding parts with the covering, neither the guiding parts nor the catheter retained thereby are directly contacted with the dressing. For this reason, it is possible to prevent the catheter from slipping out and the like due to the peeling off of the dressing.

In the fifth embodiment, the catheter fixing device of the third or fourth embodiment is provided with guiding parts which widen in the direction from one end of the catheter retention slot to the other end.

Therefore, according to the fifth embodiment when the one narrow end of the guiding parts serves as the entry side of the fixed member from which the fixed member is slid in towards the other, broader end, the guided parts can be locked into the guiding parts by, for example, accessing the fixed member obliquely from above. For this reason, it is possible to shorten the movement length for when the fixed member is slid in along the planar direction of the fixing device body.

Herein, the fixed member may be made from a material that is more rigid that the fixing device body.

Therefore, according to such a configuration, the retaining parts can be reliably deformed and thus the catheter retaining slot can be reliably narrowed because the retaining parts of the fixing device body can be efficiently compressed by means of a fixed member that is made from a rigid material. For this reason, the catheter can be clamped in and fixed with greater strength by means of the retaining parts.

The depth of the catheter retaining slot may be made to be equivalent to the diameter of the catheter.

Therefore, according to such a configuration, because the depth of the catheter retaining slot is made to be equivalent to the diameter of the catheter, the overall thickness of the fixing device body can be held in check and, in addition thereto, the total thickness of the catheter fixing device can be reliably held in check. Note that the depth of the catheter retaining slot may also be made to be larger than the diameter of the catheter.

Both the fixing device body and the fixed member may each be provided with positioning parts.

Therefore, according to such a configuration, there is a position relationship between the positioning parts provided on the fixing device body and the fixed member, such that the same can be grasped when both are in the positioned state.

As detailed above, according to embodiments 1 to 5, a catheter fixing device can be provided wherein the total thickness can be held in check while unintentional disconnection can still be prevented, without posing much burden for the patient and despite the ability to be stably fixed.

### Brief Description of the Drawings

Various embodiments of the presently disclosed catheter fixing device are described herein with reference to the drawings, wherein:
FIG. 1(a) is a plan drawing illustrating the fixing device body of the catheter fixing device in the first embodiment embodying the present invention; (b) is a frontal drawing illustrating the fixing device body; (c) is a cross-section taken along section line A-A of FIG. 1(b); (d) is a cross-section taken along section line B-B of FIG. 1(a); (e) is a cross-section taken along section line C-C of FIG. 1(a); and (f) is a cross-section taken along section line D-D of FIG. 1(a);
FIG. 2(a) is a plan drawing illustrating the fixed member of the catheter fixing device of the first embodiment; (b) is a frontal drawing illustrating the fixed member shown in FIG. 2(a); and (c) is a cross-section taken along section line E-E of FIG. 2(b).
FIG. 3(a) is a plan drawing illustrating the first embodiment of the catheter fixing device in the state wherein the fixed member has been fitted onto the fixing device body; (b) is a frontal drawing of the catheter fixing device shown in FIG. 3(d); (c) is a cross-section taken along section line F-F of FIG. 3(a); (d) is a cross-section taken along section line G-G of FIG. 3(a); and (e) is a cross-section taken along section H-H of FIG. 3(a);
FIG. 4 (a) to (c) are perspective drawings for describing the procedure for fitting the fixed member onto the fixing device body in the first embodiment;
FIG. 5(a) is a plan drawing illustrating the fixing device body of the catheter fixing device in the second embodiment; (b) is a frontal drawing illustrating the fixing device body of the catheter fixing device of the second embodiment; (c) is a perspective drawing of the fixed member viewed from below; (d) is a bottom view drawing illustrating the fixed member shown in FIG. 4(c); and (e) is a side view drawing illustrating the fixed member shown in FIG. 4(d);
FIG. 6 (a) to (c) are perspective drawings describing the procedure for fitting the fixed member onto the fixing device body in the second embodiment;
FIG. 7 (a) to (c) are side view drawings describing the procedure for fitting the fixed member onto the fixing device body in the second embodiment;
FIG. 8 (a) is a perspective drawing illustrating the third embodiment of the catheter fixing device; and (b) is a plan drawing of the third embodiment shown in FIG. 8(a);
FIG. 9(a) is a perspective drawing illustrating the catheter fixing device; and (b) is a plan drawing of the fourth embodiment shown in FIG. 9(a);
FIG. 10(a) is a perspective drawing illustrating the fifth embodiment of the catheter fixing device; and (b) is a plan drawing of the fifth embodiment shown in FIG. 10(a);
FIG. 11 (a) is a perspective drawing illustrating the catheter fixing device in another embodiment; and (b) is a plan drawing of the embodiment shown in FIG. 11(b) **Detailed Description of Embodiments**

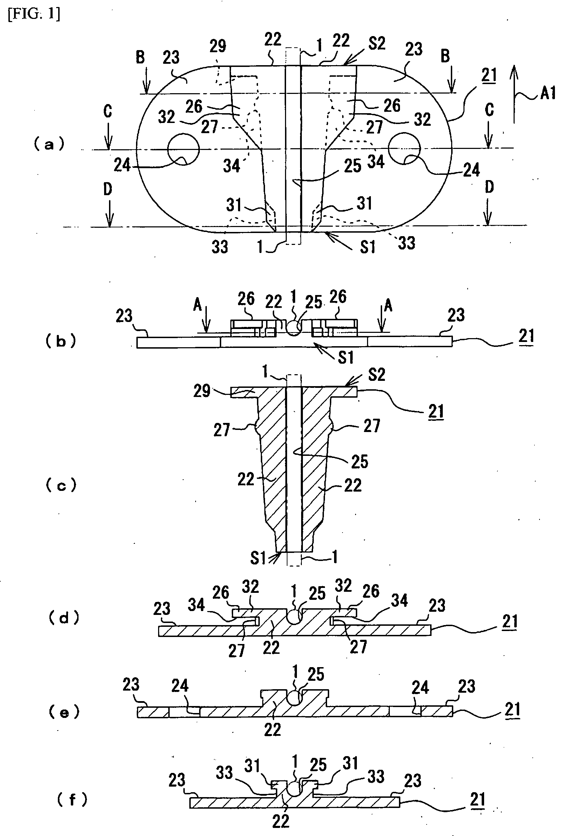

Below is a detailed description of the catheter fixing device 11 of the first embodiment embodying the present invention, based on FIG. 1 to FIG. 4.

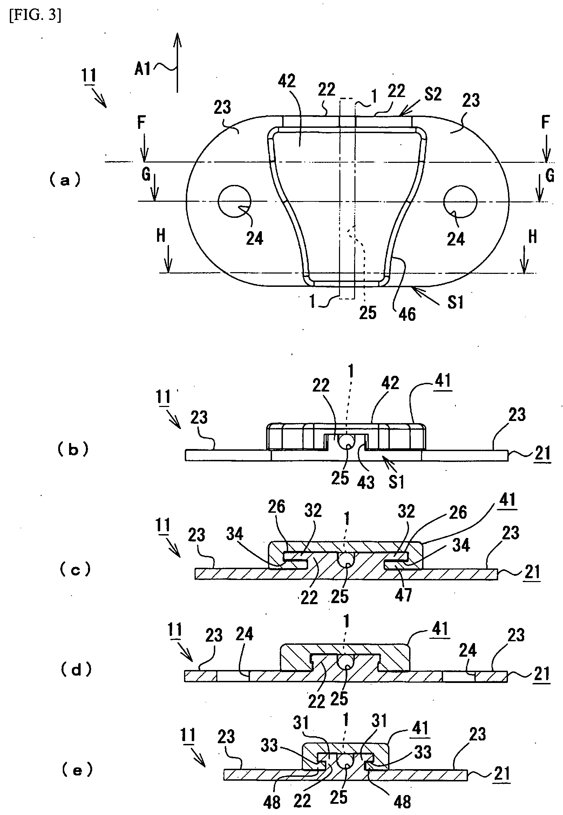

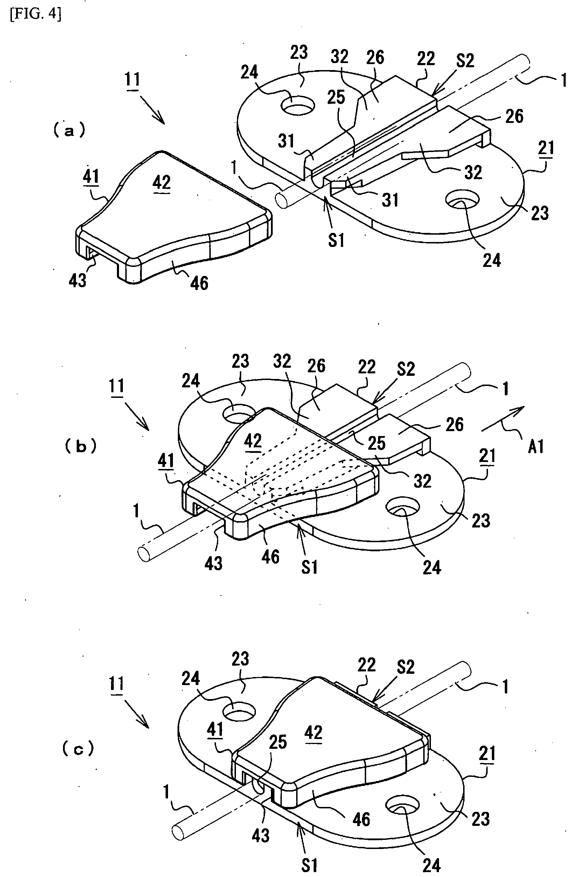

Catheter fixing device 11 of this embodiment is a medical device used when fixing a tubular catheter 1. The catheter fixing device 11 is basically constituted of two members (fixing device body 21 and covering 41 serving as the fixed member), as illustrated in FIG. 3 and FIG. 4.

As illustrated in FIG. 1, FIG. 3 and the like, fixing device body 21 is a plate-shaped member with an elliptical shape in plan view, which is formed using a comparatively soft synthetic resin material (for example, polyamide-based elastomer). In addition to polyamide-based elastomer, other soft synthetic resins may be selected, including for example polyvinyl chloride, silicone rubber, soft polyurethane, polyester-based elastomer and the like. Because the fixing device body 21 is herein the member that is directly in contact with the skin, it is preferable to use a material having flexibility and elasticity, in order to alleviate the sense of discomfort on the patient. Also, it is preferable that the synthetic resin material from which fixing device body 21 is formed be softer than the material of the tube body of catheter 1. The reason therefor is to avoid deformation of catheter 1 and collapse of the inner lumen when compressive force is applied to retain catheter 1 in fixing device body 21.

Fixing device body 21 is provided with a pair of wing pieces 23. Circular holes 24 are provided on these wing pieces 23. These holes 24 are through-holes through which a suture is to be threaded. Note that an adhesive layer for fixedly adhering fixing device body 21 onto the skin may be formed on the reverse side of fixing device body by applying an adhesive coating or adhesive sheet. A mold-release film for protecting the adhesive layer may also be adhered thereonto prior to use. Retaining parts 22 are provided projecting out at the center part of the front-side surface of fixing device body 21. A catheter retaining slot 25 capable of retaining catheter 1 is formed in the retaining parts 22. Catheter retaining slot 25 extends along the direction traversing fixing device body 21 and also opens out on the surface side of fixing device body 21. In order to be able to hold the thickness of fixing device body 21 in check, the depth of catheter retaining slot 25 is set to a dimension equivalent to the diameter of catheter 1.

As illustrated in FIG. 1, FIG. 3 and the like, retaining parts 22 are formed so as to gradually widen going from one side (fitting start side S1) to the other side (fitting end side S2). Further, stop wall 29 is arranged on the fitting end side S2 of retaining parts 22 in order to control the stop point of covering 41.

As shown in FIG. 1, FIG. 3 and the like, guiding parts 26 are provided on the topside parts of retaining parts 22 extending as a left-right pair along the planar direction of fixing device body 21 and in parallel with the pair of wing pieces 23. The guiding parts 26 are also formed so as to gradually widen going from the fitting start side S1 to the fitting end side S2. A relatively narrow first region 31 is arranged at the fitting start side S1 of guiding parts 26, and a relatively wide second region 32 is arranged at the fitting end side S2 of guiding parts 26. A first concavity 33 is formed between first region 31 of guiding parts 26 and the surface of fixing device body 21, and a second concavity 34 is formed between second region 32 of guiding parts 26 and the surface of fixing device body 21. Note that, as shown in FIG. 1(c) and (d) and the like, engaging projections 27 are provided as a left-right pair at the point at which second concavity 34 is found in retaining parts 22.

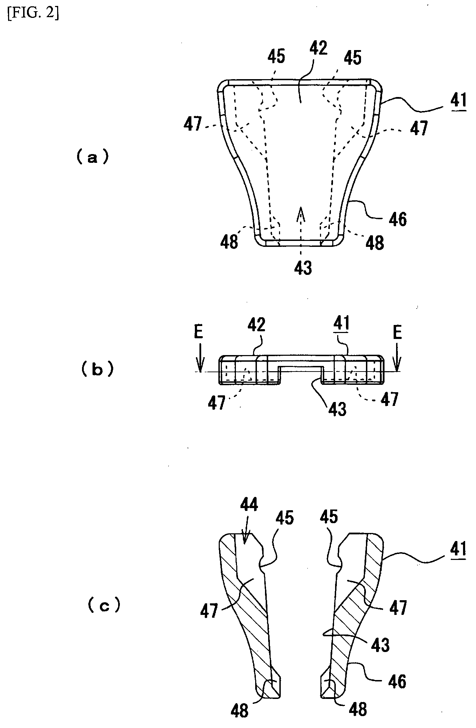

FIG. 2, FIG. 3 and the like illustrate covering 41 serving as the fixed member. Covering 41 is formed using a synthetic resin material that is more rigid than the synthetic resin material constituting fixing device body 21 (for example, polypropylene). In addition to polypropylene, other rigid synthetic resins may be selected including polyethylene, polycarbonate, polyamide, polyester, polyoxymethylene, rigid polyurethane, or ABS and the like. Covering 41 has a surface 42 and also has a reverse surface 44 arranged in contact with the upper surface of guiding parts 26. Covering 41 is sized such that guiding parts 22 are entirely covered in the state wherein the reverse surface 44 side is in contact with the upper surface of guiding parts 22.

A slot 43 extending along the longitudinal direction of the covering is formed at the center part on the reverse surface 44 side of covering 41. The slot 43 widens somewhat going from the fore of the covering to the rear. Front portion 46 of covering 41 is formed so as to be narrower than the rear portion, in order to enable easy grasping with the fingers during operation. Guided parts 47, 48, which are capable of engaging guiding parts 26, are formed as a set of two on the left and right sides interposing slot 43 on the reverse surface 44 side of covering 41. Guided parts 48, which have a relatively small surface area, are arranged at the front portion 46 of covering 41. When completely interlocked, guided parts 48 are positioned in first concavity 33 to enter a state of engagement with first region 31 of guiding parts 26 (see FIG. 3(e)). At this time, the inner edges of guided parts 48 contact the sides of retaining parts 22 from both the left and right sides. On the other hand, guided parts 48, which have a relatively large surface area, are arranged at the rear portion of covering 41. When completely fitted, guided parts 47 are positioned in second concavity 34 to enter a stage of engagement with second region 32 of guiding parts 26 (see FIG. 3(c)). At this time, the inner edges of guided parts 47 contact the sides of retaining parts 22 from both the left and right sides. Note that, as illustrated in FIG. 2(a) and (c), engaging concavities 45 are formed on the inner edges of guided parts 47 to be able to engage engaging projections 27 of guiding parts 22.

Next, a description will be provided for the procedure for fixing catheter 1 using catheter fixing device 11 of this embodiment constituted as described above, based on FIG. 4.

For example, the tip of catheter 1 is guided percutaneously into the subclavian vein, and the remainder of catheter 1 is led out of the skin from a puncture site in the precordium of the patient. Further, catheter fixing device 11 is mounted onto the part outside the body of catheter 1 that has been drawn out of the skin.

Firstly, the part outside the body of catheter 1 is inserted into catheter retention slot 25 formed in retaining parts 22 of fixing device body 21 (see FIG. 4(a)). After insertion, fixing device body is placed onto the skin. When the reverse surface side of fixing device 21 has an adhesive layer, fixing device body may be attached to the skin by means of the adhesive layer in this stage.

Next, covering 41 is arranged at fitting start side S 1 of fixing device body 21 and accesses fixing device body 21 from this position in order to be slid in a linear manner towards fitting end side S2 (that is, along planar direction A1 of the body of fixed member 21) (see FIG. 4(a) and (b)). Specifically, retaining parts 22 and guiding parts 26 are inserted from the rear side of slot 43 of covering 41. At this time, the operation is more easily done because the technician is able to grasp front portion 46 of covering 41 from both sides with his fingers. Note that while fitting start side S 1 of retaining parts 22 and guiding parts 26 is formed so as to be narrow, the rear side of the covering for slot 43 is formed so as to be broad. For this reason, retaining parts 22 and guiding parts 26 can be easily inserted into slot 43. Additionally, when covering 41 is moved horizontally, guided parts 47, 48 are installed below guided part 26 such that covering 41 is guided along guiding parts 26. Therefore, covering 41 can be smoothly slid in.

As illustrated in FIG. 4(c), when covering 41 is moved to slide up to fitting end side S2, the ends of guided parts 47 abut stop wall 29 and thus covering 41 is unable to be moved any further. Also, engaging concavities 45 on the covering 41 side reach the position of engaging projections 27 on the fixing device body 21 side such that each engages the other. At this time, the technician will feel a click at the tip of his fingers, and can thus know from the tactile sensation that the same has entered a completely fitted state. Further, in such a completely fitted state, retaining parts 22 are compressed from both the left and right sides by guided parts 47, 48, which causes retaining parts 22 to be deformed, and thus catheter retaining slot 25 is narrowed. As a result, the part of catheter 1 outside the body is clamped in by retaining parts 22 and firmly fixed into catheter retaining slot 25. Note that in the completely fitted state, reverse surface 44 of covering 41 and the upper surface of guiding parts 26 enter a state of contact, and retaining parts 22 and guiding parts 26 also enter a state nearly entirely covered by covering 41. Further, catheter fixing device 11 and the puncture part may thereafter be protected by the application of a dressing as needed.

Also, when the fixation by catheter fixing device 11 is released and catheter 1 is removed, the operation is the inverse of when fitting as described above. When a dressing has been applied, firstly the dressing is peeled off, thus exposing catheter fixing device 11. While force is thereupon easily applied to the upward direction of catheter fixing device 11, on the other hand force is not easily applied to the direction that would remove covering 41, and therefore the risk that covering 41 would be taken off along with the dressing is extremely small. Note that guided parts 47, 48 serve not only to engage guiding parts 26, but also to prevent removal of covering 41. Next, covering 41 is moved by being slid in the direction of fitting end side S1 to thus separate covering 41 from fixing device body 21. Catheter retaining slot 25, which had been narrowed by the compression of retaining parts 22, is thereupon widened, thus making it possible to release the fixation of catheter 1 and remove catheter 1.

Therefore, the following effects are achieved according to this embodiment.
(1) According to the configuration of catheter fixing device 11 of this embodiment, when covering 41 is slid in to fit with fixing device body 21, compressive force is less likely to be applied in the vertical direction onto the skin. Accordingly, catheter 1 can be stably fixed without such a great burden on the patient. Moreover, force is less prone to be applied to the direction that would remove covering 41 even when the dressing is peeled off, and therefore an accidental event like the removal of covering 41 therewith is unlikely to occur.
(2) In addition, in conventional catheter fixing devices, if one intends to hold the total thickness in check while employing the conventional design, there is no allowance for providing a clamping part in the height direction of the covering, such that often fixation is inadequate, but there is no need according to the configuration of this embodiment to provide a clamping part in such a direction. Accordingly, even when covering 41 is provided with a structure such as a clamping part, the total thickness of catheter fixing device 11 can be held in check (that is, a low profile is achieved), and also catheter 1 can be prevented from falling out.
(3) Also, according to catheter fixing device 11 of this embodiment, the depth of catheter retaining slot 25 is made to be equivalent to the diameter of catheter 1. However, reverse surface 44 of covering 41 and the upper surface of guiding parts 26 enter a state of contact. Accordingly, the overall thickness of fixing device body 21 can be held in check, and in addition thereto the total thickness of catheter fixing device 11 can be reliably held in check. Therefore, catheter fixing device 11 is no longer unwieldy and is thus less likely to give a sense of discomfort at the point of fixation. Additionally, because catheter fixing device 11 does not become unwieldy even when protected by the application of a dressing, air bubbles are unlikely to occur in the periphery thereof. Accordingly, the point between the dressing and catheter fixing device 11 is less likely to become a hotbed for infection.
(4) In this embodiment, because covering 41 is made from a synthetic resin material that is more rigid than that of fixing device body 21, retaining parts 22, made from a comparatively softer synthetic resin material, can be effectively compressed. Therefore, retaining parts 22 can be reliably deformed so as to reliably narrow catheter retention slot 25. For this reason, catheter 1 is more strongly clamped in and fixed by means of retaining parts 22. Also, in this embodiment, using a synthetic resin material and not a metal material for both fixing device body 21 and covering 41 is advantageous in that there is no particular need for removal when undergoing tests that use x-rays.

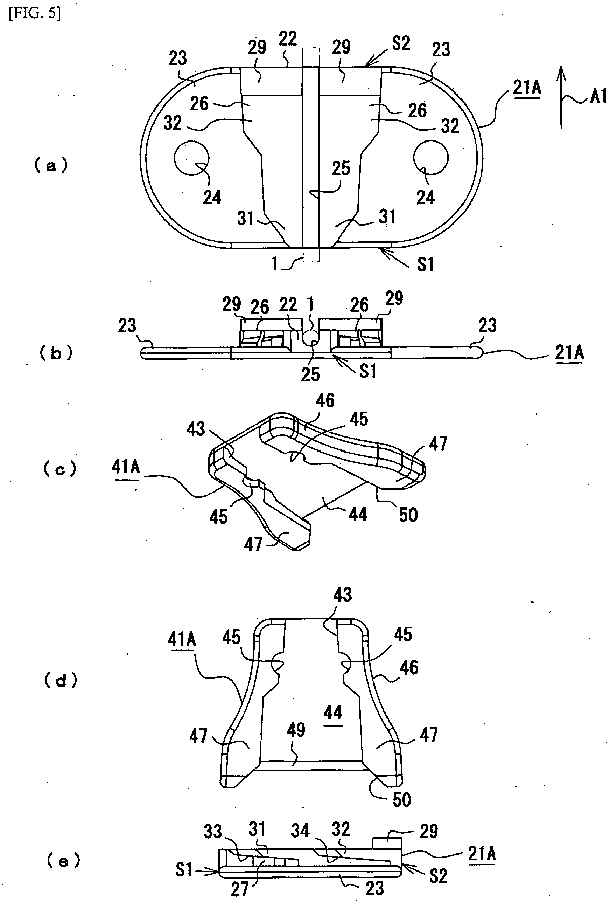

Below is a detailed description of the catheter fixing device 11A of the second embodiment embodying the present invention, based on FIG. 5 to FIG. 7. Herein, the description primarily focuses on the points of difference from the first embodiment, and a description for points in common is forgone as the same numerals are given to the same members.

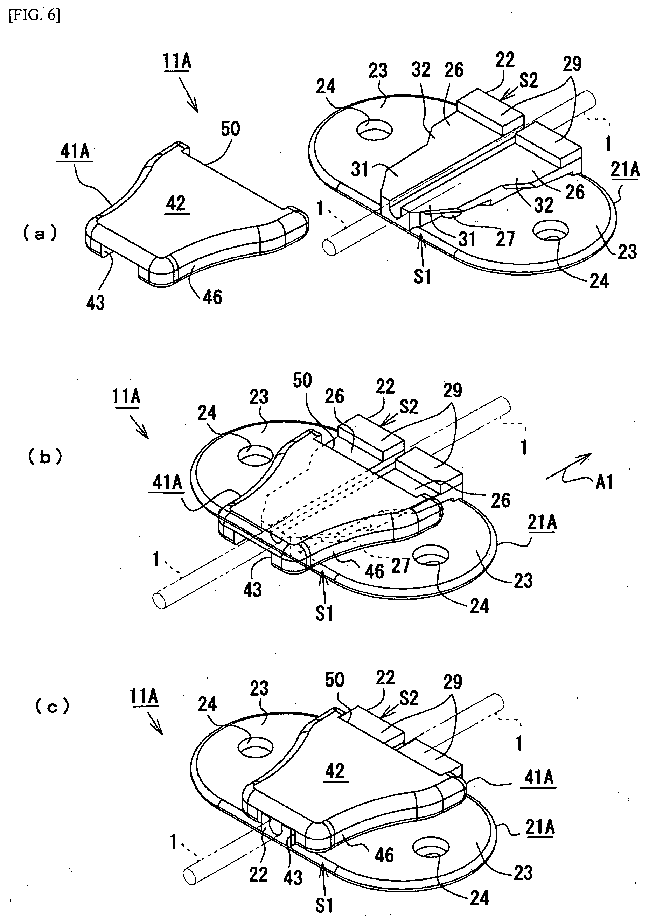

Catheter fixing device 11A of this embodiment is basically constituted of two members (fixing device body 21 A and covering 41 A) as illustrated in FIG. 5 and FIG. 6. Stop wall 29 in this fixing device body 21A is larger than in the previous embodiment, and is formed so as to project out beyond the top surface of guide parts 26. Fitting concavity 50 into which stop wall 29 can be fitted is formed at the rear part of covering 41A. Therefore, in the case illustrated in FIG. 6(c), stop wall 29 is fitted into fitting concavity 50 such that it can be visually understood when there is a completely fitted state.

As illustrated in FIG. 5(e), FIG. 6(a), FIG. 7(a) and the like, engaging projections 27 are provided as a left-right pair at the point where first concavity 33 is found on the side of retaining parts 22. Also, as illustrated in FIG. 5(c) and (d) and the like, engaging concavities 45 which can engage with the engaging projections of guiding parts 22 are formed on the inner edges of guided parts 47 on covering 41A. In this embodiment, both engaging projections 27 and engaging concavities 45 are arranged at positions closer to the fore. Therefore, a more reliable fixation can be ensured than when, as in the previous embodiment, engaging projections 27 and engaging concavities 45 are arranged at positions closer to the rear. That is, although in the previous embodiment engaging projections 27 and engaging concavities 45 will engage even when, for example, the front end of covering 41 A is loose, neither will be engaged in this embodiment.

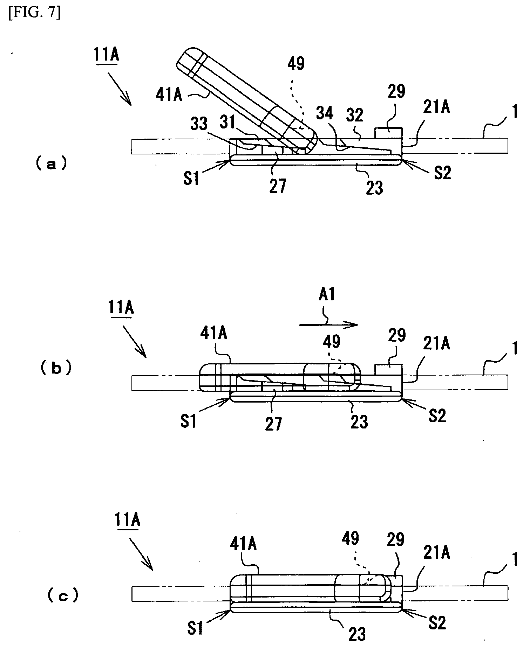

As illustrated in FIG. 5(a) and the like, guiding parts 26 widen going from one end (fitting start side S1) of catheter retaining slot 25 to the other end (fitting end side S2). Therefore, when the one narrow end of guiding parts 26 serves as the entry side of covering 41A from which covering 41A is slid in towards the other, broad end, covering 41A can be accessed obliquely from above. For this reason, it is possible to shorten the movement length for when covering 41A is slid in along the planar direction of fixing device body 21 A. Therefore, the space required for operation can be reduced. Note that the upper surface of first concavities 33 and second concavities 34 are beveled in order to easily access from above in such a manner. Accordingly, as illustrated in FIG. 5(e) and the like, first concavities 33 and second concavities 34 are formed so as to narrow from fitting end side S2 substantially upward. Also, as illustrated in FIG. 5(d) and the like, sloped surface 49 sloping at a smaller angle than perpendicular toward reverse surface 44 of covering 41A is formed at the rear end of covering 41A. When there is this sloped surface 49, it is possible to more smoothly access obliquely from above (see FIG. 7(a)).

Note that the embodiments of the present invention may be modified as follows.

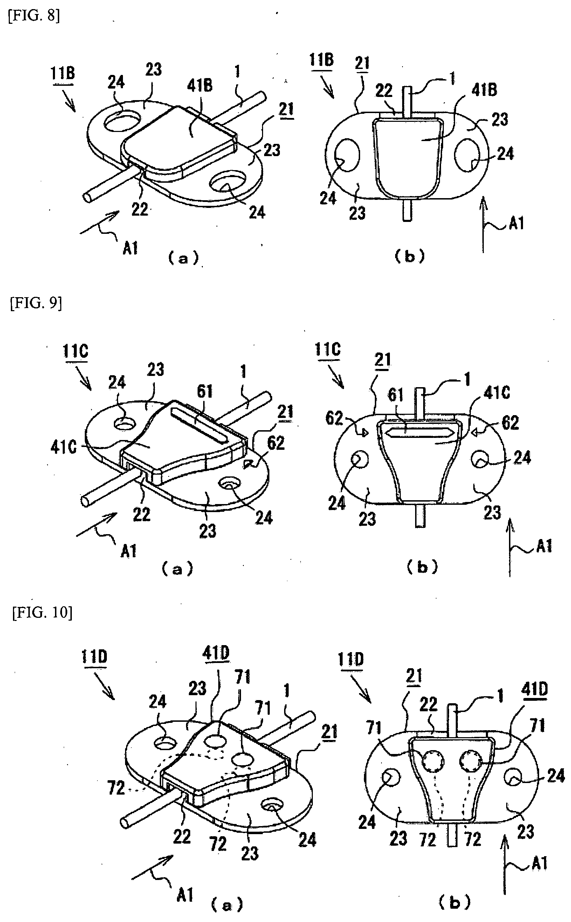

For example, as in catheter fixing device 11B of the third embodiment illustrated in FIG. 8(a) and (b), covering 41B may be used, wherein front portion 46 is not quite as thin. Also, hole 24 may not be a circular shape, but may rather be, for example, an elliptical shape.

For example, as in catheter fixing device 11C of the fourth embodiment illustrated in FIG. 9(a) and (b), a structure may be provided for mutual positioning. That is, triangular-shaped positioning mark 62 is provided serving as a positioning part at two points on the surface of fixing device body 21. On the other hand, belt-shaped position mark 61 extending in the direction orthogonal to catheter retention slot 25 is provided on the surface of covering member 41C. Therefore, according to such a configuration, it can be sensed when there is a completely fitted state when positioning marks 61, 62 provided on fixing device body 21 and covering 41C form a positional relationship aligned on a straight line.

For example, as in catheter fixing device 11D of a fifth embodiment illustrated in FIG. 10(a) and (b), positioning structures may be utilized. That is, circular window parts 71 serving as positioning parts are formed on covering 41 D in catheter fixing device 11D. Window parts 71 may be through-holes, and may also be transparent parts. On the other hand, circular positioning marks 72 serving as positioning parts are formed at two points on the upper surface of guiding parts 26 provided on fixing device body 21. Therefore, according to such a configuration, it can be sensed when there is a completely fitted state when positioning marks 72 form a visible positional relationship through window parts 71.

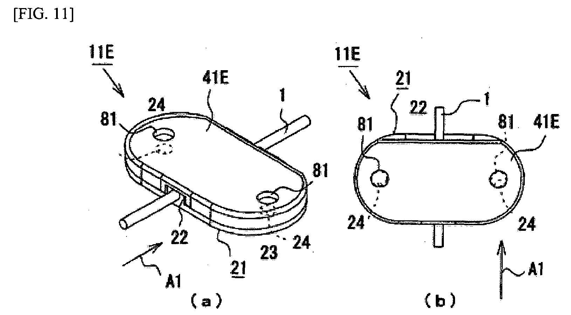

For example, positioning structures as in catheter fixing device 11E of another embodiment illustrated in FIG. 11 (a) and (b) may be used. That is, circular window parts 81 serving as positioning parts are formed as a left-right part on covering 41 E in catheter fixing device 11E. However, the position thereof corresponds to the position of a pair of holes 24 in fixing device body 21E. In other words, herein a pair of holes 24 are used as the positioning parts. Therefore, according to such a configuration, it can be sensed when there is a completely fitted state when holes 24 form a visible positional relationship through window parts 81. Note that in this embodiment, covering 41E has substantially the same shape and size as fixing device body 21E. Therefore, according to such a configuration, it is possible not to have a difference in levels on the surface of catheter fixing device 11E.

In the embodiments above, covering 41 is intended to be made from an opaque resin material, but may also be made from a transparent resin material.

In the above embodiments, catheter retention slot 25 opens out on the surface side of fixing device body 21, but may also take a configuration that opens on the reverse side.

Next, the following is a list of technical concepts to be understood by means of the embodiments described above.
(1) In any of the embodiments described above, the fixing device body and fixed member may be made from resin.
(2) In any of the embodiments described above, the fixed member may be made from an opaque resin material.
(3) In any of the embodiments described above, the retaining parts may be provided with engaging projections which can engage with engaging concavities formed on the guided parts of the fixed member when the fixed member is completely fitted.
(4) In any of the embodiments described above, the fixed member may be slid in in a linear manner along the lengthwise direction of the catheter (the direction along which the catheter retention slot extends).
(5) In any of the embodiments described above, the fixed member may be made from a more rigid material than that of the fixing device body.
(6) In any of the embodiments described above, the depth of the catheter retention slot may be equal to or greater than the diameter of the catheter.
(7) In any of the embodiments described above, the fixing device body and the fixed member may be provided with positioning parts.

Persons skilled in the art will understand that the devices specifically described herein and illustrated in the accompanying drawings are non-limiting exemplary embodiments. It is envisioned that the elements and features illustrated or described in connection with one exemplary embodiment may be combined with the elements and features of another without departing from the scope of the present disclosure. As well, one skilled in the art will appreciate further features and advantages of the disclosure based on the above-described embodiments. Accordingly, the disclosure is not to be limited by what has been particularly shown and described, except as indicated by the appended claims.

## Claims

1. A catheter fixing device (11) for fixing a tubular catheter (1) comprising: a plate-shaped fixing device body (21) having retaining parts (22) and a pair of wing pieces (23) that form a catheter retention slot (25), and a fixed member (41) that can be fitted onto the fixing device body (21) by being slid along a planar direction of the fixing device body (21), wherein the retaining parts (22) are compressed by the fixed member (41) when the fixed member (41) is fitted onto the fixing device body (21) so as to narrow the catheter retention slot (25).

2. The catheter fixing device (11) according to Claim 1, wherein a surface side of the fixing device body (21) is provided with guiding parts (26) that extend along the planar direction of the fixing device body (21), and the fixed member (41) is provided with guided parts (47,48) that can be locked into the guiding parts (26).

3. The catheter fixing device (11) according to Claim 2, wherein the catheter retention slot (25) opens out on the surface side of the fixing device body (21), and wherein the guiding parts (26) are provided as a left-right pair on the retaining parts (22), such that the retaining parts (22) are compressed from both the left and the right by the guided parts (47,48) when the fixed member (41) is fitted onto the fixing device body (21) so as to narrow the catheter retention slot (25).

4. The catheter fixing device (11) according to claims 2 or 3, wherein the fixed member (41) is a covering that entirely covers the guiding parts (47,48) in a state wherein a reverse side of the fixed member (41) is made to contact a surface of the guiding parts (47,48).

5. The catheter fixing device (11) according to any one of claims 2-4, wherein the guiding parts (47,48) widen in a direction from one end of the catheter retention slot (25) to the other end.

## Patentansprüche

1. Katheterfixierungsvorrichtung (11) zur Fixierung eines Schlauchkatheters (1), umfassend: einen plattenförmigen Fixierungsvorrichtungskörper (21), der Halteteile (22) und ein Paar Flügelteile (23) aufweist, die einen Katheterhalteschlitz (25) bilden, und ein festes Element (41), das auf den Fixierungsvorrichtungskörper (21) montiert werden kann, indem es entlang einer planaren Richtung des Fixierungsvorrichtungskörpers (21) geschoben wird, wobei die Halteteile (22) durch das feste Element (41) zusammengedrückt werden, wenn das feste Element (41) auf den Fixierungsvoruchtungskörper (21) montiert wird, so dass der Katheterhalteschlitz (25) verengt wird.

2. Katheterfixierungsvorrichtung (11) nach Anspruch 1, wobei eine Oberflächenseite des Fixierungsvorrichtungskörpers (21) mit Führungsteilen (26) ausgestattet ist, die sich entlang der planaren Richtung des Fixierungsvomichtungskörpers (21) erstrecken, und das feste Element (41) mit geführten Teilen (47, 48) ausgestattet ist, die in die Führungsteile (26) geklemmt werden können.

3. Katheterfixierungsvorrichtung (11) nach Anspruch 2, wobei der Katheterhalteschlitz (25) in die Oberflächenseite des Fixierungsvorrichtungskörpers (21) mündet und wobei die Führungsteile (26) als Links-/Rechts-Paar auf den Halteteilen (22) vorgesehen sind, so dass die Halteteile (22) sowohl von links als auch von rechts durch die geführten Teile (47, 48) zusammengedrückt werden, wenn das feste Element (41) auf den Fixierungsvorrichtungskörper (21) montiert wird, so dass der Katheterhalteschlitz (25) verengt wird.

4. Katheterfixierungsvorrichtung (11) nach Anspruch 2 oder 3, wobei das feste Element (41) eine Abdeckung ist, die die Führungsteile (47, 48) in einem Zustand, bei dem eine Rückseite des festen Elements (41) in Kontakt mit einer Oberfläche der Führungsteile (47, 48) gebracht ist, vollständig abdeckt.

5. Katheterfixierungsvorrichtung (11) nach einem der Ansprüche 2-4, wobei die Führungsteile (47, 48) in eine Richtung von einem Ende des Katheterhalteschlitzes (25) zum anderen Ende breiter werden.

## Revendications

1. Dispositif de fixation de cathéter (11) destiné à fixer un cathéter tubulaire (1) comprenant : un corps de dispositif de fixation en forme de plaque (21) comportant des parties de retenue (22) et une paire de pièces en forme d'ailettes (23) qui forment une fente de retenue de cathéter (25), et un élément fixe (41) qui peut être monté sur le corps de dispositif de fixation (21) en étant glissé le long d'une direction plane du corps de dispositif de fixation (21), dans lequel les parties de retenue (22) sont comprimées par l'élément fixe (41) lorsque l'élément fixe (41) est monté sur le corps de dispositif de fixation (21) de sorte à réduire la fente de retenue de cathéter (25).

2. Dispositif de fixation de cathéter (11) selon la revendication 1, dans lequel un côté de surface du corps de dispositif de fixation (21) est doté de parties de guidage (26) qui s'étendent le long de la direction plane du corps de dispositif de fixation (21), et l'élément fixe (41) est doté de parties guidées (47, 48) qui peuvent être verrouillées dans les parties de guidage (26).

3. Dispositif de fixation de cathéter (11) selon la revendication 2, dans lequel la fente de retenue de cathéter (25) s'ouvre sur le côté de surface du corps de dispositif de fixation (21), et dans lequel les parties de guidage (26) sont prévues sous forme d'une paire gauche-droite sur les parties de retenue (22), de sorte que les parties de retenue (22) sont comprimées à la fois depuis la gauche et la droite par les parties guidées (47, 48), lorsque l'élément fixe (41) est monté sur le corps de dispositif de fixation (21) de sorte à réduire la fente de retenue de cathéter (25).

4. Dispositif de fixation de cathéter (11) selon les revendications 2 ou 3, dans lequel l'élément fixe (41) est un revêtement qui recouvre entièrement les parties de guidage (47, 48) dans un étant dans lequel un côté inverse de l'élément fixe (41) est mis en contact avec une surface des parties de guidage (47, 48).

5. Dispositif de fixation de cathéter (11) selon l'une quelconque des revendications 2 à 4, dans lequel les parties de guidage (47, 48) s'élargissent dans une direction allant d'une extrémité à l'autre de la fente de retenue de cathéter (25).
